# EUROPÄISCHE PATENTSCHRIFT

(11) **EP 1 973 423 B1**
(45) Veröffentlichungstag und Bekanntmachung des Hinweises auf die Patenterteilung: **15.03.2017**
(21) Anmeldenummer: 06829109.5
(22) Anmeldetag: 23.11.2006
(51) Int. Cl.: A23L 27/30, A23L 33/10, A61K 9/20, A61K 31/7016, A61K 31/353, A23L 2/00, A23L 2/39, A23L 2/52, A23L 33/105, A23F 3/16, A23L 27/00

(54) **PRÄPARAT ENTHALTEND EINE POLYPHENOLHALTIGE ZUSAMMENSETZUNG UND ISOMALTULOSE**
FORMULATION COMPRISING A POLYPHENOL-CONTAINING COMPOSITION AND ISOMALTULOSE
PRÉPARATION CONTENANT UNE COMPOSITION POLYPHÉNOLIQUE ET DE L'ISOMALTULOSE

(30) Priorität: 25.11.2005 DE 102005056652
(43) Veröffentlichungstag der Anmeldung: 01.10.2008
(73) Patentinhaber: Südzucker Aktiengesellschaft Mannheim/Ochsenfurt, 68165 Mannheim (DE); DSM IP Assets B.V., 6411 TE Heerlen (NL)
(72) Erfinder: DÖRR, Tillmann, 67591 Hohen-Sülzen (DE); HAUSMANNS, Stephan, 65185 Wiesbaden (DE); KOWALCZYK, Jörg, 67304 Eisenberg/Steinborn (DE); BECKER, Martina, 79540 Lörrach (DE); KILLEIT, Ulrich, 79639 Grenzach-Wyhlen (DE)
(74) Vertreter: Schrell, Andreas
(86) Internationale Anmeldenummer: PCT/EP2006/011219
(87) Internationale Veröffentlichungsnummer: WO 2007/059953

(56) Entgegenhaltungen:
- EP-A1- 1 421 859
- WO-A1-2004/008870
- WO-A2-2005/013720
- JP-A- 2000 069 945
- US-A1- 2003 180 432
- US-A1- 2004 219 141
- DATABASE WPI Week 200264 Derwent Publications Ltd., London, GB; AN 2002-591760 XP002423784 & CN 1 352 902 A (YANG S) 12. Juni 2002 (2002-06-12)

## Beschreibung

Die vorliegende Erfindung betrifft Präparate, enthaltend polyphenolhaltige Zusammensetzungen und Isomaltulose, Verwendungen des vorgenannten Präparats sowie die Verwendung von Isomaltulose als geschmacksmaskierendes Agens in einem eine polyphenolhaltige Zusammensetzung enthaltenden Präparat.

In einer beachtlichen Vielzahl von Nahrungs- und Genussmitteln sowie insbesondere in Getränken finden sich eine Reihe von teilweise erwünschten, teilweise aber auch unerwünschten Bitterstoffen, also Verbindungen, die beim Konsumenten, insbesondere dem menschlichen Konsumenten, einen bitteren Geschmack verursachen. Typische Bitterstoffe sind zum Beispiel Substanzen aus der Stoffklasse der Glykoside, aber auch der Isoprenoide und Catechine, die als Grundsubstanz einer Vielzahl von Oligomer- oder Polymer-Gerbstoffen anzutreffen sind. Bitterstoffe auf Polyphenolbasis sind auch in Tees oder Teeextrakten vorhanden. Zu derartigen Extrakten gehören auch Extrakte des grünen Tees, zum Beispiel der auch als TEAVIGO® bezeichnete Extrakt aus Blättern des grünen Tees, der mindestens 90 % Epigallocatechingallat (EGCG) enthält. Obgleich dieser bereits einen vergleichsweise akzeptablen Grad an Bitterkeit aufweist, ist er für einige Konsumenten noch zu bitter. Gerbstoffe wie Tanninsäuren weisen in der Regel einen bitteren Geschmack auf, der in Getränken und Nahrungs-, Genuss- oder Arzneimitteln störend sein kann. Das US-Patent 5,902,628 beschreibt die Verkürzung eines anhaltenden süßen Nachgeschmacks des Intensivsüßstoffes Sucralose durch Beimischung von Tanninsäuren. Obgleich die Tanninsäure gemäß dieser Druckschrift den als nachteilig angesehenen stark süßen Nachgeschmack der Sucralose positiv beeinflusst, erweist sich dennoch der bittere Geschmack der Tanninsäure in vielen Anwendungsfällen trotz gleichzeitiger Anwesenheit der Sucralose als nachteilig.

Die natürlich, zum Beispiel in Honig, vorkommende reduzierende Disaccharid-Ketose Isomaltulose (6-O-α-D-Glucopyranosyl-fructose), die dem Fachmann auch als Palatinose® bekannt ist, wird hauptsächlich als Ausgangsstoff zur Herstellung von Isomalt, eines nahezu äquimolaren Gemisches der Diastereomere 6-O-α-D-Glucopyranosyl-D-sorbit (1,6-GPS) und 1-O-α-D-Glucopyranosyl-D-mannit (1,1-GPM) eingesetzt. Als süßendes Agens wird Isomaltulose aufgrund seiner geringen Süßkraft und des daraus resultierenden Geschmacks hauptsächlich in Kombination mit Zuckeraustauschstoffen und/oder Süßstoffen in Lebensmitteln eingesetzt. Aufgrund des erst im Dünndarmbereich erfolgenden verzögerten Abbaus der Isomaltulose wird diese auch in speziellen Lebensmitteln für Sportler eingesetzt, um den oxidativen Metabolismus aufrechtzuerhalten.

Isomaltulose kristallisiert in Form eines Monohydrats. Die Löslichkeit von Isomaltulose in Wasser beträgt 0,49 g wasserfreie Isomaltulose pro g Wasser. Isomaltulose weist vorteilhafte akariogene Eigenschaften auf, da sie von der menschlichen Mundflora kaum abgebaut wird. Isomaltulose wird lediglich von den Glucosidasen der menschlichen Dünndarmwand verzögert gespalten, wobei die resultierenden Abbauprodukte Glucose und Fructose resorbiert werden. Dies resultiert, verglichen zu schnell verdaulichen Kohlenhydraten, in einem langsamen Anstieg der Blutglucose. Isomaltulose benötigt im Unterschied zu schnell verdaulichen, hochglykämischen Lebensmitteln kaum Insulin zur Verstoffwechslung.

Teilweise wird Isomaltulose auch eingesetzt, um den unangenehmen Geschmack anderer Lebensmittel zu überdecken. In der WO 2004/008870 sind Instant-Tees beschrieben, die Isomaltulose als alleiniges Süßungsmittel und als alleinigen Trägerstoff enthalten. Die EP 0 809 939 A1 beschreibt einen Milchsäurebakterien und Bifidobakterien enthaltenden Joghurt, der raffiniertes Fischöl mit einem hohen Anteil ungesättigter Fettsäuren sowie ein süßendes Agens, beispielsweise Isomaltulose enthält. Die Zugabe von Isomaltulose soll die Entwicklung des typischen Fisch-Geschmackes und Fisch-Geruches verhindern. Die JP 63152950 A2 beschreibt die Herstellung von Gemüse-Gelee-Erzeugnissen unter Verwendung von Gemüsearten und einem Gelierungsmittel, wobei Isomaltulose und andere Zusätze wie Zimt eingesetzt werden, um den unangenehmen Geruch von Gemüsebestandteilen zu überdecken.

Die DE 690 005 48 T2 beschreibt Sucralose und Isomaltulose enthaltende Süßstoffzusammensetzungen, wobei diese Zusammensetzung einen synergistischen Effekt aufweisen, dass heißt eine größere Süßkraft zeigen, als dies durch eine einfache Addition der von den Süßmittelkomponenten gezeigten Süßkraft erwartet würde. Das Süßungsmittelgemisch kann beispielsweise zur Herstellung von Getränken und Süßwaren eingesetzt werden.

Nach wie vor besteht jedoch ein Bedarf an einer Lehre, gemäß der der unerwünschte bittere Geschmack von Bitterstoffen, insbesondere Bitterstoffen auf Polyphenolbasis, in Präparaten, also zum Beispiel Lebensmitteln, Getränken, Arzneimitteln oder Genussmitteln, überlagert und so möglichst der Wahrnehmung des Konsumenten entzogen werden kann.

Unter Geschmack wird hier der chemische Sinn des Menschen zur Wahrnehmung und Unterscheidung von Nahrungsstoffen verstanden. Der Mensch kann im wesentlichen vier Geschmacksqualitäten unterscheiden: süß, sauer, bitter und salzig. Der Geschmacksreiz kommt durch die Aktivierung einer Geschmackssinneszelle durch die Anlagerung von Molekülen eines Geschmacksstoffes an Rezeptormoleküle zustande. Ein Geschmacksreiz kann sowohl in seiner Qualität, beispielsweise ob er süß, sauer, bitter, salzig oder eine Mischung davon ist, in seiner Intensität, also der Geschmacksstärke, als auch in seiner Dauer unterschiedlich ausgeprägt sein.

Der vorliegenden Erfindung liegt daher das technische Problem zugrunde, eine Lehre bereitzustellen, gemäß der der unerwünschte bittere Geschmack von Bitterstoffen, insbesondere polyphenolhaltigen Zusammensetzungen, in einem Präparat maskiert wird, das heißt vom Konsumenten, insbesondere menschlichen oder tierischen Konsumenten, nicht mehr oder nur stark reduziert wahrgenommen wird, insbesondere ein bitterer Geschmackseindruck während und nach des Verzehrs nicht mehr wahrgenommen wird.

Die Erfindung löst das ihr zugrunde liegende technische Problem durch die Bereitstellung der Lehre, Isomaltulose einzusetzen, um den bitteren Geschmack polyphenolhaltiger Gemische, insbesondere Zusammensetzungen, zu maskieren. Die Erfindung löst das ihr zugrundeliegende Problem insbesondere auch durch die Bereitstellung eines Präparates, enthaltend eine polyphenolhaltige Zusammensetzung und Isomaltulose wie in den Ansprüchen definiert.

Die Erfindung betrifft also die Lehre, Isomaltulose zusammen mit einer, insbesondere bitter schmeckenden, polyphenolhaltigen Zusammensetzung einzusetzen, wobei die Isomaltulose den ansonsten bitteren Geschmack der polyphenolhaltigen Zusammensetzung maskiert. Erfindungsgemäß wirkt die Isomaltulose also als geschmacksmaskierendes Agens, welches sowohl während des Verzehrs der polyphenolhaltigen Zusammensetzung beziehungsweise eines eine polyphenolhaltige Zusammensetzung enthaltenden Präparats wirkt, als auch den bitteren Geschmack, der ansonsten auch noch nach dem Verzehr vom Konsumenten empfunden wird, also der Nachgeschmack, maskiert. Vorteilhafterweise kann dadurch ein Präparat bereitgestellt werden, das aufgrund der verwendeten Isomaltulose Süßkraft aufweist, gleichwohl aber zahnschonend, akariogen, nicht laxativ und Diabetiker geeignet ist.

Im Zusammenhang mit der vorliegenden Erfindung wird unter einer Geschmacksmaskierung einer polyphenolhaltigen Zusammensetzung verstanden, dass ein von sachverständigen Testpersonen als bitter empfundener Geschmack der polyphenolhaltigen Zusammensetzung in einem Vergleichspräparat mit polyphenolhaltiger Zusammensetzung und Vergleichssüßungsmittel mit statistisch relevanter Aussagekraft in signifikant reduziertem Ausmaß, vorzugsweise gar nicht mehr, wahrgenommen wird, wenn diese polyphenolhaltige Zusammensetzung in einem Präparat der Erfindung statt mit dem Vergleichssüßungsmittel zusammen mit Isomaltulose vorliegt.

Im Zusammenhang mit der vorliegenden Erfindung wird unter einer bitter schmeckenden, polyphenolhaltigen Zusammensetzung eine Zusammensetzung verstanden, die Polyphenole, insbesondere Polyphenolderivate, in einer Menge enthält, die von sachverständigen Testpersonen mit statistisch relevanter Aussagekraft als bitter schmeckend angesehen wird.

Die polyphenolhaltige Zusammensetzung der vorliegenden Erfindung ist eine Zusammensetzung, die mindestens 90 Gew.-% Epigallocatechingallat, im Folgenden auch als EGCG (Polyphenol(-)-epigallocatechin-3-gallat) bezeichnet, aufinreist, vorzugsweise mindestens 94 Gew.-% EGCG , jeweils bezogen auf die Trockensubstanz der polyphenolhaltigen Zusammensetzung.

Die vorliegende Erfindung betrifft ein Präparat nach Anspruch 1, enthaltend 0,1 bis 10 Gew.% polyphenolhaltige Zusammensetzung, bezogen auf die Trockensubstanz des Präparats, mit einem Anteil von mindestens 90 Gew.% and Epigallocatechingallat (EGCG), vorzugsweise 94 Gew.-% an EGCG, bezogen auf die Trockensubstanz der polyphenolhaltigen Zusammensetzung, und 30 bis 80 Gew. % Isomaltulose, bezogen auf die Trockensubstanz des Präparates.

In einer weiteren bevorzugten Ausführungsform betrifft die Erfindung ein vorgenanntes Präparat, wobei die polyphenolhaltige Zusammensetzung einen Anteil von höchstens 2,5 Gew.-% an Coffein, vorzugsweise von höchstens 0,1 Gew.-% an Coffein, jeweils bezogen auf das Gewicht der Trockensubstanz der polyphenolhaltigen Zusammensetzung, aufweist.

In einer weiteren bevorzugten Ausführungsform betrifft die vorliegende Erfindung ein vorgenanntes Präparat, wobei die polyphenolhaltige Zusammensetzung einen Anteil von höchstens 2,5 Gew.-% an Gallocatechingallat (GCG), bezogen auf das Gewicht der Trockensubstanz der polyphenolhaltigen Zusammensetzung, aufinreist.

Die Erfindung betrifft in einer weiteren bevorzugten Ausführungsform ein Präparat der vorgenannten Art, wobei die polyphenolhaltige Zusammensetzung einen Anteil von höchstens 5,0 Gew.-% an Epicatechingallat (ECG), vorzugsweise von höchstens 3,0 Gew.-% ECG, jeweils bezogen auf das Gewicht der Trockensubstanz der polyphenolhaltigen Zusammensetzung, aufweist.

Die Erfindung betrifft in einer weiteren bevorzugten Ausführungsform ein vorgenanntes Präparat, wobei die polyphenolhaltige Zusammensetzung einen Anteil von höchstens 0,1 Gew.-% an Gallussäure, bezogen auf das Gewicht der Trockensubstanz der polyphenolhaltigen Zusammensetzung, aufweist.

Die Erfindung betrifft ferner in einer weiteren bevorzugten Ausführungsform ein Präparat der vorgenannten Art, wobei die polyphenolhaltige Zusammensetzung, abgesehen von EGCG, einen Anteil von höchstens 5,0 Gew.-% an sonstigen Polyphenolen und Catechinen wie Gallocatechingallat (GCG), Catechingallat (CG), Epicatechingallat (ECG), Epigallocatechin (EGC), Gallocatechin (GC) und Epicatechin (EC), jeweils bezogen auf das Gewicht der Trockensubstanz der polyphenolhaltigen Zusammensetzung, aufweist. In bevorzugter Ausführungsform ist die polyphenolhaltige Zusammensetzung TEAVIGO®.

TEAVIGO® ist ein hochaufgereinigter Extrakt von Blättern des grünen Tees Camellia sinensis und weist mindestens 90 Gew.-%, vorzugsweise mindestens 94 Gew.-% EGCG und maximal 0,1 Gew.-% Coffein auf.

Die Erfindung betrifft darüber hinaus in einer weiteren bevorzugten Ausführungsform natürlich auch die Verwendung von Isomaltulose in einem eine polyphenolhaltige Zusammensetzung enthaltenden Präparats zur Geschmacksmaskierung. In besonders bevorzugter Ausführungsform ist die Geschmacksmaskierung eine Geschmacksmaskierung einer polyphenolhaltigen Zusammensetzung.

In einer weiteren bevorzugten Ausführungsform der vorliegenden Erfindung betrifft diese die Verwendung eines Präparats der vorliegenden Erfindung zur Erhöhung der Fettverbrennung in Säugetieren, insbesondere in Menschen. In einer weiteren bevorzugten Ausführungsform betrifft die vorliegende Erfindung die Verwendung eines vorgenannten Präparates zur Unterstützung des Fettmetabolismus in Säugetieren, insbesondere in Menschen.

In einer weiteren bevorzugten Ausführungsform der vorliegenden Erfindung betrifft die Erfindung die Verwendung eines vorgenannten Präparates zur Reduzierung der Fettmasse in Säugetieren, insbesondere in Menschen.

In einer weiteren bevorzugten Ausführungsform der vorliegenden Erfindung betrifft diese die Verwendung eines vorgenannten Präparates zur Herstellung eines Arzneimittels zur Erhöhung der Fettverbrennung und/oder zur Unterstützung des Fettmetabolismus und/oder zur Reduzierung der Fettmasse in Säugetieren, insbesondere im Menschen.

Die Erfindung betrifft darüber hinaus auch ein Instant-Pulver, enthaltend 50 bis 80 Gew.-% Isomaltulose, 0,1 bis 10 Gew.-% EGCG, 0,1 bis 10 Gew.-% Coffein sowie 0,005 bis 1,0 Gew.-% B-Vitamine.

Im Zusammenhang mit der vorliegenden Erfindung werden unter Säugetieren insbesondere Menschen, Katzen, Hunde und Pferde verstanden. Im Zusammenhang mit der vorliegenden Erfindung werden und B-Vitaminen insbesondere Vitamin B₁, Vitamin B₂ und Nicotinamid verstanden.

In bevorzugter Ausführungsform wird EGCG in den erfindungsgemäßen Präparaten und Verwendungen so verwendet, dass die effektive Dosis von EGCG von 0,14 bis 25 mg/kg Körpergewicht/Tag, bevorzugt von 2,0 bis 9,0 mg/kg Körpergewicht/Tag, besonders bevorzugt von 4,0 bis 9,0 mg/kg Körpergewicht/Tag und insbesondere von 4,0 bis 4,5 mg/kg Körpergewicht/Tag beträgt.

In bevorzugter Ausführungsform der vorliegenden Erfindung ist die polyphenolhaltige Zusammensetzung ein Pflanzenextrakt, insbesondere ein isolierter und aufgereinigter, insbesondere hochrein aufgereinigter Pflanzenextrakt, insbesondere ein Teeextrakt.

In einer weiteren bevorzugten Ausführungsform ist der Extrakt ein Extrakt aus schwarzem Tee, aus Oolong-Tee oder aus grünem Tee, vorzugsweise ein Extrakt aus Blättern des grünen Tees, nämlich Camellia sinensis.

In einer weiteren bevorzugten Ausführungsform enthält der Extrakt der vorgenannten Art zusätzlich natürliche oder naturidentische Geruchs- und/oder Geschmacksstoffe.

Gemäß der Erfindung ist vorzugsweise vorgesehen, dass die Isomaltulose verwendet wird, um den bitteren Geschmack der in einem Präparat der vorliegenden Erfindung vorhandenen polyphenolhaltigen Zusammensetzung zu maskieren, das heißt unkenntlich oder nicht beziehungsweise stark reduziert wahrnehmbar zu machen. In bevorzugter Ausführungsform der vorliegenden Erfindung ist ein derartiges Präparat eine für den menschlichen oder tierischen Verzehr bestimmte oder geeignete Zusammensetzung, zum Beispiel ein Getränkepulver, ein Instantpulver, ein Produkt für die orale Hygiene, ein kosmetisches Produkt, eine Trockenaromaformulierung, ein Lebens-, Nahrungs- oder Genussmittel oder ein Futtermittel. In bevorzugter Ausführungsform kann dieses Präparat in trockener, vorzugsweise rieselfähiger Form vorliegen, insbesondere in agglomerierter, pulverisierter und/oder gefriergetrockneter Form.

Die Erfindung betrifft insbesondere auch, gegebenenfalls beschichtete, Präparate, die in Form von Komprimaten, Kapseln oder Tabletten als Nahrungs-, Genuss-, Lebens- oder Arzneimittel, ein Instantpulver, als Getränkepulver, als Getränke oder als kosmetische sowie Produkte für die orale Hygiene vorliegen.

Selbstverständlich kann das Präparat auch eingesetzt werden, um Getränke herzustellen, das heißt als Instantpulver, insbesondere Instant-Teepulver oder als Trockenaromaformulierung zu dienen. Die erfindungsgemäß beobachtende Effekte finden sich dann in dem mittels des erfindungsgemäßen Präparates hergestellten Getränk, zum Beispiel einem Teegetränk, welches ebenfalls Gegenstand der vorliegenden Lehre ist.

In einer weiteren bevorzugten Ausführungsform können in dem erfindungsgemäßen Präparat zusätzlich lebensmittelverträgliche Säuren und/oder Salze vorhanden sein. In einer weiteren bevorzugten Ausführungsform der Erfindung können in dem erfindungsgemäßen Präparat auch Intensivsüßstoffe, zum Beispiel Saccharin, Saccharin-Na, Saccharin-K, Saccharin-Ca, Natriumcyklamat, Calziumcyklamat, Acesulfam-K, Aspartam, Dulcin, Steviosid, Neohesperedin-Dihydrochalkon oder Sucralose vorhanden sein.

In einer weiteren bevorzugten Ausführungsform können in dem Präparat Antioxidantien, Stabilisatoren, Mineralstoffe und/oder Spurenelemente vorhanden sein. In einer weiteren bevorzugten Ausführungsform können in dem erfindungsgemäßen Präparat auch Vitamine vorhanden sein, zum Beispiel natürliche oder synthetische Vitamine, insbesondere Vitamin A, Vitamin Vitamin B₂, Vitamin B₃, Vitamin B₅, Vitamin B₆, Vitamin B₁₂, Vitamin B-Komplex, Nicotinamid, Vitamin C, Vitamin E, Vitamin F und Vitamin K.

In einer weiteren bevorzugten Ausführungsform können die erfindungsgemäßen Präparate Fließhilfsmittel enthalten, zum Beispiel Siliziumdioxid.

In einer weiteren bevorzugten Ausführungsform können die erfindungsgemäßen Präparate natürliche und/oder synthetische Farbstoffe enthalten, zum Beispiel Farbstoffe pflanzlicher Herkunft, tierischer Herkunft, anorganische Pigmente, Produkte der enzymatischen Bräunung, Produkte der nicht-enzymatischen Bräunung und Kohlenhydraterhitzungsprodukte. Als synthetische Farbstoffe können beispielsweise Azo-Verbindungen, Triphenylmethan-Verbindungen, Indigoid-Verbindungen, Xanthen-Verbindungen oder Chinolin-Verbindungen eingesetzt werden.

In einer weiteren bevorzugten Ausführungsform betrifft die vorliegende Erfindung ein vorgenanntes Präparat, wobei der Anteil der polyphenolhaltigen Zusammensetzung an dem Präparat 0,1 bis 10 Gew.-%, vorzugsweise 0,3 bis 10 Gew.-%, insbesondere 0,3 bis 5 Gew.-%, vorzugsweise 0,6 bis 9 Gew.-%, insbesondere 0,7 bis 8 Gew.-%, bevorzugt 0,9 bis 7 Gew.-%, insbesondere 1 bis 5 Gew.-% (jeweils bezogen auf Trockensubstanz am Präparat) beträgt.

In einer weiteren bevorzugten Ausführungsform betrifft die vorliegende Erfindung ein vorgenanntes Präparat, wobei der Anteil der Isomaltulose am Präparat vorzugsweise 30 bis 85 Gew.-%, vorzugsweise 30 bis 80 Gew.-%, vorzugsweise 50 bis 80 Gew.-%, insbesondere 35 bis 75 Gew.-%, insbesondere 40 bis 70 Gew.-%, vorzugsweise 45 bis 65 Gew.-%, insbesondere 50 bis 61 Gew.-% (jeweils bezogen auf Trockensubstanz des Präparats) beträgt.

In einer weiteren bevorzugten Ausführungsform betrifft die vorliegende Erfindung ein vorgenanntes Präparat, wobei der Anteil der lebensmittelverträglichen Säuren am Präparat 5 bis 10 Gew.-% (bezogen auf Trockensubstanz des Präparats) beträgt.

In einer weiteren bevorzugten Ausführungsform betrifft die vorliegende Erfindung ein vorgenanntes Präparat, wobei der Anteil der Intensivsüßstoffe am Präparat 0,1 bis 1 Gew.-% (bezogen auf Trockensubstanz des Präparats) beträgt.

In einer weiteren bevorzugten Ausführungsform betrifft die vorliegende Erfindung ein vorgenanntes Präparat, wobei der Anteil der Geschmacksstoffe am Präparat 0,5 bis 30 Gew.-% (bezogen auf Trockensubstanz des Präparats) beträgt.

In einer weiteren bevorzugten Ausführungsform weist das erfindungsgemäße Präparat abgesehen von Isomaltulose keinen weiteren Zucker, insbesondere keine Saccharose, keine Fructose und/oder keine Glucose auf. Vorzugsweise ist die Isomaltulose der einzige und alleinig in dem Präparat vorkommende Zucker, insbesondere das einzige und alleinig in dem Präparat vorkommende süßende Agens.

Im Zusammenhang mit der vorliegenden Erfindung wird unter dem Begriff süßendes Agens eine Substanz verstanden, die Süßkraft aufweist und zum Beispiel Lebensmitteln oder Getränken zugesetzt wird, um einen süßen Geschmack hervorzurufen. Im Zusammenhang mit der vorliegenden Erfindung werden die süßenden Agenzien unterteilt in Zucker, wie Isomaltulose, Saccharose, Glucose oder Fructose, die Körper und Süßkraft geben sowie "Süßungsmittel", also Stoffe, die keine Zucker sind, aber trotzdem Süßkraft aufweisen, welche wiederum untergliedert werden in "Zuckeraustauschstoffe", also süßende Agenzien, die einen Körper und einen physiologischen Brennwert zusätzlich zu einer Süßkraft aufweisen (körpergebendes Süßungsmittel) und "Intensivsüßstoffe", also Stoffe, die in der Regel eine sehr hohe Süßkraft, aber keinen Körper und in der Regel keinen oder nur einen geringfügigen physiologischen Brennwert aufweisen.

In einer besonders bevorzugten Ausführungsform ist das erfindungsgemäße Präparat zahnschonend, akariogen und/oder diätetisch.

Erfindungsgemäß kann allerdings in einer weiteren Ausführungsform vorgesehen sein, dass die Isomaltulose zusammen mit Intensivsüßstoffen und/oder gegebenenfalls auch Zuckeraustauschstoffen, zum Beispiel Isomalt, in dem Präparat verwendet wird.

In einer weiteren bevorzugten Ausführungsform betrifft die Erfindung die erfindungsgemäßen polyphenolhaltigen Präparate, enthaltend Isomaltulose zusammen mit mindestens einem, vorzugsweise stark süßenden, Kohlenhydrat, das vorzugsweise eine äquivalente Süßkraft von > 0,5, bezogen auf eine 10%ige Saccharoselösung, aufweist, z.B. Saccharose, Fructose, Glucose, Invertzucker und/oder Oligofructose. Die Erfindung betrifft auch erfindungsgemäße polyphenolhaltige Präparate, enthaltend Isomaltulose und Glucosehaltige, Fructose-haltige und/oder Leucrose-haltige Sirupe, z.B Sucromalt. Die Erfindung betrifft auch erfindungsgemäße polyphenolhaltige Präparate, enthaltend Isomaltulose und Mischungen von Saccharose, Glucose und/oder Fructose mit anderen Kohlenhydraten.

Die stark maskierenden sensorischen Eigenschaften der Isomaltulose, insbesondere in Kombination mit stark süßenden Kohlenhydraten, liefern in Polyphenol-haltigen, insbesondere in Teavigo-haltigen Getränkeformulierungen im Vergleich zu klassisch aufgesüßten Produkten ein ausgewogenes und abgerundetes Süßeprofil mit vollem Mundgefühl.

Insbesondere zeigen die erfindungsgemäß bevorzugten polyphenolhaltigen Präparate mit einem Kohlenhydratanteil, insbesondere Isomaltulose-Anteil, von 30 bis 85%, bevorzugt 45 bis 65%, in Getränken einen ausgesprochenen positiven sensorischen Gesamteindruck.

Weitere vorteilhafte Ausgestaltungen der vorliegenden Erfindung ergeben sich aus den Untersprüchen.

Die Erfindung wird anhand eines Ausführungsbeispiels näher erläutert.

### Beispiel

**Rezeptur 1 (Vergleichsrezeptur)**

| **Komponenten** | **Instantpulver (g/kg)** | **rtd (g/200 ml)** |
|---|---|---|
| Maltodextrin | 569,300 | 8,540 |
| Zitronensäure, wasserfreies Pulver | 66,670 | 1,000 |
| Trinatriumcitrat, Dihydratpulver | 12,500 | 0,188 |
| TWINSWEET® | 3,330 | 0,050 |
| Grapefruitgeschmack | 8,330 | 0,125 |
| TEAVIGO® | 2,540 | 0,038 |
| Coffein | 3,330 | 0,050 |
| CustoMix BE-P | 0,670 | 0,010 |
| Grapefruit 250L-Pulver | 333,330 | 5,000 |
| Gesamt | 1000,000 | 15,000 |

**Rezeptur 2 mit Saccharose (Vergleichsrezeptur)**

| **Komponenten** | **Instantpulver (g/kg)** | **rtd (g/200 ml)** |
|---|---|---|
| Saccharose | 607,800 | 6,078 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGO® | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

**Rezeptur 3 mit Isomaltulose**

| **Komponenten** | **Instantpulver (g/kg)** | **rtd (g/200 ml)** |
|---|---|---|
| Isomaltulose | 607,800 | 6,078 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGO® | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

**Rezeptur 4 mit Isomaltulose**

| **Komponenten** | **Instantpulver (g/kg)** | **rtd (g/250 ml)** |
|---|---|---|
| Isomaltulose | 583,300 | 7,000 |
| Zitronensäure, wasserfreies Pulver | 83,300 | 1,000 |
| Trinatriumcitrat, Dihydratpulver | 15,000 | 0,180 |
| TWINSWEET® | 4,200 | 0,050 |
| Grapefruitgeschmack | 11,500 | 0,138 |
| TEAVIGO® | 3,200 | 0,038 |
| Coffein | 4,200 | 0,050 |
| CustoMix BE-P | 0,800 | 0,010 |
| Grapefruit 250L-Pulver | 294,500 | 3,534 |
| Gesamt | 1000,000 | 12,000 |

**Rezeptur 5 mit Isomaltulose und Saccharose**

| **Komponenten** | **Instantpulver (g/kg)** | **rtd (g/200 ml)** |
|---|---|---|
| Isomaltulose | 407,800 | 4,078 |
| Saccharose | 200,000 | 2,000 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGO® | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

**Rezeptur 6 mit Isomaltulose und Fructose**

| **Komponenten** | **Instantpulver (g/kg)** | **rtd (g/200 ml)** |
|---|---|---|
| Isomaltulose | 457,800 | 4,578 |
| Fructose | 150,000 | 1,500 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGOO | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

**Rezeptur 7 mit Isomaltulose und Trockenglucose**

| **Komponenten** | **Instantpulver (g/kg)** | **rtd (g/200 ml)** |
|---|---|---|
| Isomaltulose | 207,800 | 2,078 |
| Trockenglucose(Fa. Agrana) | 400,000 | 4,000 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGO® | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

**Rezeptur 8 mit Isomaltulose/Invertzuckersirup-Compound**

| **Komponenten** | **Instantpulver (g/kg)** | **rtd (g/200 mol)** |
|---|---|---|
| Isomaltuloseanteil im Compound | 487,800 | 4,878 |
| Invertzuckeranteil im Compound | 120,000 | 1,200 |
| Zitronensäure, wasserfreies Pulver | 80,000 | 0,800 |
| Trinatriumcitrat, Dihydratpulver | 14,400 | 0,144 |
| TWINSWEET® | 3,000 | 0,030 |
| Grapefruitgeschmack | 11,000 | 0,110 |
| TEAVIGO® | 3,800 | 0,038 |
| Coffein | 4,000 | 0,040 |
| CustoMix BE-P | 1,000 | 0,010 |
| Grapefruit 250L-Pulver | 275,000 | 2,750 |
| Gesamt | 1000,000 | 10,000 |

*Die Herstellung des Compounds erfolgte durch Agglomeration, wobei fein vermahlene Isomaltulose eingesetzt wurde und die leicht verdünnte Invertzuckerlösung (etwa gleiche Teile Saccharose, Glucose und Fructose) mit einem TS von ca. 60% als Binderkomponente aufgesprüht wurde.

Aus den vorgenannten Rezepturen ergibt sich aus der Spalte "Instantpulver" die Konzentration der jeweils angegebenen Rezepturkomponente in g/kg im Gesamtpräparat. Aus der Spalte "rtd" (ready to drink, trinkfertig) ergibt sich die angegebene Konzentration der Rezepturkomponente in Gramm pro 200 ml in einer mit dem Präparat hergestellten Getränk. Für die Vergleichsrezeptur 1 wurden pro 200 ml Getränk 15 g des Instantpulvers eingesetzt, während in Rezeptur 2, 3, 5 bis 8 von dem erfindungsgemäßen Instantpulver 10 g pro 200 ml Getränk und in Rezeptur 4 12 g pro 250 ml Getränk eingesetzt wurden.

TEAVIGO® ist ein hochaufgereinigter Extrakt von Blättern des grünen Tees Camellia sinensis und weist mindestens 90 Gew.-%, vorzugsweise mindestens 94 Gew.-% EGCG und maximal 0,1 Gew.-% Caffein auf.

Zur Herstellung des Getränkes wurde das Präparat in Wasser unter Umrühren trinkfertig gelöst.

Das Getränk wurde einem geschulten Panel von 15 Probanden zur Verkostung vorgesetzt.

Die Probanden sollten im Rahmen einer Profilanalyse mit Skala die Bitterkeit (beim Trinken und Nachgeschmack), den Süßeeindruck, das Mundgefühl, den Aromaeindruck und den Gesamteindruck der aus den Rezepturen hergestellten Getränke beurteilen.

Die Attribute wurden auf einer Skala von 0 bis 5 wie folgt definiert:
Bitterkeit (beim Trinken): 0 = unangenehm bitter; 5 = Bitternote kaum wahrnehmbar
Bitterkeit (Nachgeschmack): 0 = unangenehm bitter; 5 = Bitternote kaum wahrnehmbar
Süßeeindruck: 0 = unausgewogen; 5 = ausgewogen, rund
Mundgefühl: 0 = unangenehm; 5 = vollmundig, angenehm
Aromaeindruck: 0 = kurz, flach ; 5 = langanhaltend, ausgewogen
Gesamteindruck: 0 = unausgewogen, qualitativ schlecht; 5 =ausgewogen, rund, qualitativ gut

Die Probanden stellten in statistisch aussagekräftiger Weise für die Vergleichsrezeptur sowohl während des Verkostens als auch unmittelbar danach einen deutlich bittereren Geschmack als für die erfindungsgemäße Rezepturen fest. Weitere Vorteile zeigten sich auch bei den anderen überprüften Attributen. Insbesondere bei der Süße und dem Gesamteindruck zeigen die erfindungsgemäßen Produkte mit den Isomaltulose-Kohlenhydratmischungen sehr positive sensorische Eigenschaftsprofile. Die Ergebnisse der Profilanalyse sind numerisch in der Tabelle zusammengefasst.

Diese Ergebnisse sind überraschend, da die Rezepturen hinsichtlich der Süßkraft variieren. Die Süßkraft ist offensichtlich nicht der zur Kaschierung der Bitternote verantwortliche Parameter, das heisst um die gleiche Menge an Bitterstoffen, insbesondere an polyphenolhaltigem Extrakt, also TEAVIGO®, zu überdecken. Die Daten zeigen, dass Isomaltulose zusammen mit einer polyphenolhaltigen Zusammensetzung ein besonders positives sensorisches Profil bewirkt. Wird Isomaltulose in Kombination mit mindestens einem stark süßenden Kohlenhydrat, zum Beispiel Fructose, Glucose, Saccharose und/oder Invertzucker verwendet, ergibt sich ein nochmals verbessertes, positives, sensorisches Profil.

**Tabelle: Ergebnisse der sensorischen Profilanalyse**

| | Rezeptur 1 Maltodextrin | Rezeptur 2 Saccharose | Rezeptur 3 Isomaltulose | Rezeptur 4 Isomaltulose | Rezeptur 5 Isom./Sacch. | Rezeptur 6 Isom./Fruc. | Rezeptur 7 Isom./Trglu. | Rezeptur 8 Isom./Invertz. |
|---|---|---|---|---|---|---|---|---|
| Bitterkeit beim Trinken | 1.6 | 1,9 | 3,1 | 3,2 | 3,2 | 3,1 | 3,0 | 3,5 |
| Bitterkeit (Nachgeschmack) | 2,0 | 2.3 | 3,9 | 3,8 | 4,2 | 4,3 | 4,3 | 4,4 |
| Süßeeinduck | 2,4 | 3,1 | 2,9 | 2,7 | 3,5 | 3,8 | 3,3 | 3,9 |
| Mundgefühl | 1,5 | 2,7 | 2,9 | 3,1 | 3,2 | 3,4 | 3,2 | 3,5 |
| Aromaeindruck | 3.1 | 3.2 | 3,7 | 3,6 | 3,8 | 4,0 | 3,7 | 4,1 |
| Gesamteindruck | 1,9 | 2,6 | 3,3 | 3,2 | 3,6 | 3,8 | 3,5 | 4,0 |

## Patentansprüche

1. Präparat, enthalten 0,1 bis 10 Gew. % polyphenolhaltige Zusammensetzung (bezogen auf Trockensubstanz des Präparats) mit einem Anteil von mindestens 90 Gew. % an Epigallocatechingallat (EGCG), bezogen auf die Trockensubstanz der polyphenolhaltigen Zusammensetzung, und 30 bis 80 Gew. % Isomaltulose, bezogen auf die Trockensubstanz des Präparates.

2. Präparat nach Anspruch 1, wobei die polyphenolhaltige Zusammensetzung einen Anteil von mindestens 94 Gew.-% an EGCG, bezogen auf das Gewicht der Trockensubstanz der polyphenolhaltigen Zusammensetzung, aufweist.

3. Präparat nach Anspruch 1 oder 2, wobei die polyphenolhaltige Zusammensetzung einen Anteil von höchstens 2.5 Ges.-% an Coffein, vorzugsweise von höchstens 0.1 Gew..-%, jeweils bezogen auf das Gewicht der Trockensubstanz der polyphenolhaltigen Zusammensetzung, aufweist.

4. Präparat nach einem der vorhergehenden Ansprüche, wobei die polyphenolhaltige Zusammensetzung einen Anteil von höchstens 2.5 Gew.-% an Gallocatechingallat (GCG), bezogen auf das Gewicht der Trockensubstanz der polyphenolhaltigen Zusammensetzung, aufweist.

5. Präparat nach einem der vorhergehenden Ansprüche, wobei die polyphenolhaltige Zusammensetzung einen Anteil von höchstens 5.0 Gew.-% an Epicatechingallat (ECG), vorzugsweise von höchstens 3.0 Gew.-%, jeweils bezogen auf das Gewicht der Trockensubstanz der polyphenolhaltigen Zusammensetzung, aufweist.

6. Präparat nach einem der vorhergehenden Ansprüche, wobei die polyphenothaltige Zusammensetzung einen Anteil von höchstens 0.1 Gew.-% an Gallussäure, bezogen auf das Gewicht der Trockensubstanz der polyphenolhaltigen Zusammensetzung, aufweist.

7. Präparat nach einem der vorhergehenden Ansprüche, wobei die polyphenolhaltige Zusammensetzung, abgesehen von EGCG, einen Anteil von höchstens 5.0 Gew.-% an sonstigen Polyphenolen und Catechinen wie Gallocatechingallat (GCG), Catechingallat (CG), Epicatechingallat (ECG), Epigallocatechin (EGC), Gallocatechin (GC) und Epicatechin (EC), bezogen auf das Gewicht der Trockensubstanz der polyphenolhaltigen Zusammensetzung, aufweist.

8. Präparat nach einem der vorhergehenden Ansprüche, wobei das Präparat neben der Isomaltulose mindestens ein stark süßendes Kohlenhydrat aufweist.

9. Präparat nach Anspruch 8, wobei der Anteil der Isomaltulose, und des stark süßenden Kohlenhydrats am Präparat 30 bis 85 Gew.-%, bezogen auf das Gewicht der Trockensubstanz des Präparates, beträgt.

10. Verwendung eines Präparats nach einem oder mehreren der Ansprüche 1 bis 9 zur Erhöhung der Fettverbrennung in Säugetieren, insbesondere in Menschen.

11. Verwendung eines Präparats nach einem oder mehreren der Ansprüche 1 bis 9 zur Unterstützung des Fettmetabolismus in Säugetieren, insbesondere in Menschen.

12. Verwendung eines Präparats nach einem oder mehreren der Ansprüche 1 bis 9 zur Reduzierung der Fettmasse in Säugetieren, insbesondere in Menschen.

13. Verwendung von Isomaltulose in einem eine polyphenolhaltige Zusammensetzung enthaltenden Präparat, insbesondere einem Präparat nach einem der Ansprüche 1 bis 9 zur Geschmacksmaskierung.

14. Verwendung nach Anspruch 13, wobei die Geschmacksmaskierung eine Geschmacksmaskierung einer polyphenolhaltigen Zusammensetzung ist.

15. Verwendung nach einem der Ansprüche 10 bis 14, wobei die polyphenolhaltige Zusammensetzung ein Pflanzenextrakt ist.

16. Verwendung nach einem der Ansprüche 10 bis 15, wobei der Pflanzenextrakt ein Extrakt aus schwarzem Tee, ein Extrakt aus Oolong-Tee oder ein Extrakt aus grünem Tee ist.

17. Verwendung nach einem der Ansprüche 10 bis 16, wobei der Extrakt zusätzliche natürliche oder naturidentische Geruchs- und/oder Geschmacksstoffe enthält.

18. Verwendung nach einem der Ansprüche 10 bis 17, wobei die potyphenothaftige Zusammensetzung Epigallocatechingallat (EGCG) ist.

19. Verwendung nach einem der Ansprüche, 10 bis 18, wobei das EGCG in der polyphenolhaltigen Zusammensetzung in einem Anteil von mindestens 94 Gew.-%, jeweils bezogen auf die Trockensubstanz der polyphenolhaltigen Zusammensetzung, vorliegt.

20. Verwendung nach einem der Ansprüche 10 bis 19, wobei der Anteil der polyphenolhaltigen Zusammensetzung am Präparat 0,3 bis 5 Gew.-%, bezogen auf die Trockensubstanz des Präparats, beträgt.

21. Verwendung nach einem der Ansprüche 10 bis 20, wobei der Anteil der Isomaltulose am Präparat 40 bis 70 Gew.-%, bezogen auf die Trockensubstanz des Präparats, beträgt.

22. Verwendung nach einem der Ansprüche 10 bis 21, wobei der Anteil der Isomaltulose am Präparat 50 bis 61 Gew.-% bezogen auf die Trockensubstanz des Präparats, beträgt.

23. Verwendung nach einem der Ansprüche 10 bis 22, wobei das Präparat ein Instantpulver, eine Trockenaromaformulierung, ein Getränkepulver, ein Nahrungs-, Genuss- oder Arzneimittel ist.

24. Verwendung nach einem der Ansprüche 10 bis 23, wobei das Präparat lebensmittelverträgliche Säuren, lebensmittelverträgliche Salze, Intensivsüßstoffe, Geschmacks-, Aroma- oder Geruchsstoffe, ein weiteres süßendes Agens und/oder Coffein enthält.

25. Verwendung nach einem der Ansprüche 10 bis 24, wobei der Anteil der lebensmittelverträglichen Säuren am Präparat 5 bis 10 Gew.-%, bezogen auf die Trockensubstanz des Präparats, beträgt.

26. Verwendung nach einem der Ansprüche 10 bis 25, wobei der Anteil der intensivsüßstoffe am Präparat 0,1 bis 1 Gew.-% , bezogen auf die Trockensubstanz des Präparats, beträgt.

27. Verwendung nach einem der Ansprüche 10 bis 26, wobei der Anteil der Geschmacksstoffe am Präparat 0,5 bis 30 Gew.-%, bezogen auf die Trockensubstanz des Präparats, beträgt.

28. Verwendung nach einem der Ansprüche 10 bis 27, wobei das Präparat in trockener Form vorliegt..

29. Verwendung nach einem der Ansprüche 10 bis 28, wobei das Präparat in gefriergetrockneter, agglomerierter oder pulverisierter Form vorliegt.

30. Verwendung nach einem der Ansprüche 10 bis 29, wobei das Präparat Fließhilfsmittel, insbesondere Siliziumdioxid enthält.

31. Verwendung nach einem der Ansprüche 10 bis 30, wobei das Präparat natürliche und/oder synthetische Farbstoffe enthält.

32. Verwendung nach einem der Ansprüche 10 bis 31, wobei das Präparat natürliche oder synthetische Vitamine enthält.

33. Verwendung nach einem der Ansprüche 10 bis 32, wobei das Präparat Mineralstoffe und Spurenelemente enthält.

34. Instantpulver enthaltend 50 bis 80 Gew..-% Isomaltulose, 0.1 bis 10 Gew,-% EGCG, 0.1 bis 10 Gew.-% Coffein, sowie 0.005 bis 1.0 Gew.-% B-Vitamine.

## Claims

1. Formulation containing 0.1 to 10% by weight polyphenol-containing composition (based on the dry matter of the formulation) having a fraction of at least 90% by weight of epigallocatechin gallate (EGCG), based on the dry matter of the polyphenol-containing composition, and 30 to 80% by weight isomaltulose, based on the dry matter of the formulation.

2. Formulation according to claim 1, wherein the polyphenol-containing composition has a fraction of at least 94% by weight of EGCG, based on the weight of the dry matter of the polyphenol-containing composition.

3. Formulation according to claim 1 or 2, wherein the polyphenol-containing composition has fraction of at most 2.5% by weight of caffeine, preferably at most 0.1 % by weight, in each case based on the weight of the dry matter of the polyphenol-containing composition.

4. Formulation according to any one of the preceding claims, wherein the polyphenol-containing composition has a fraction of at most 2.5% by weight of gallocatechin gallate (GCG), based on the weight of the dry matter of the polyphenol-containing composition.

5. Formulation according to any one of the preceding claims, wherein the polyphenol-containing composition has a fraction of at most 5.0% by weight of epicatechin gallate (ECG), preferably at most 3.0% by weight, in each case based on the weight of the dry matter of the polyphenol-containing composition.

6. Formulation according to any one of the preceding claims, wherein the polyphenol-containing composition has a fraction of at most 0.1% by weight of gallic acid, based on the weight of the dry matter of the polyphenol-containing composition.

7. Formulation according to any one of the preceding claims, wherein the polyphenol-containing composition, apart from EGCG, has a fraction of at most 5.0% by weight of other polyphenols and catechins such as gallocatechin gallate (GCG), catechin gallate (CG), epicatechin gallate (ECG), epigallocatechin (EGC), gallocatechin (GC) and epicatechin (EC), based on the weight of the dry matter of the polyphenol-containing composition.

8. Formulation according to any one of the preceding claims, wherein the formulation, in addition to the isomaltulose, has at least one strongly sweetening carbohydrate.

9. Formulation according to claim 8, wherein the fraction of isomaltulose and the strongly sweetening carbohydrate of the formulation is 30 to 85% by weight, based on the weight of the dry matter of the formulation.

10. Use of a formulation according to one or more of claims 1 to 9 for increasing fat burning in mammals, in particular in humans.

11. Use of a formulation according to one or more of claims 1 to 9 for supporting fat metabolism in mammals, in particular in humans.

12. Use of a formulation according to one or more of claims 1 to 9 for reducing the fat mass in mammals, in particular in humans.

13. Use of isomaltulose in a formulation containing a polyphenol-containing composition, in particular a formulation according to one of claims 1 to 9 for taste masking.

14. Use according to claim 13, wherein the taste masking is taste masking of a polyphenol-containing composition.

15. Use according to any one of claims 10 to 14, wherein the polyphenol-containing composition is a plant extract.

16. Use according to any one of claims 10 to 15, wherein the plant extract is an extract of black tea, an extract of Oolong tea or an extract of green tea.

17. Use according to any one of claims 10 to 16, wherein the extract contains additional natural or nature-identical odour and/or taste substances.

18. Use according to any one of claims 10 to 17, wherein the polyphenol-containing composition is epigallocatechin gallate (EGCG).

19. Use according to any one of claims 10 to 18, wherein the EGCG is present in the polyphenol-containing composition in a fraction of at least 94% by weight, in each case based on the dry matter of the polyphenol-containing composition.

20. Use according to any one of claims 10 to 19, wherein the fraction of polyphenol-containing composition in the formulation is 0.3 to 5% by weight, based on the dry matter of the formulation.

21. Use according to any one of claims 10 to 20, wherein the fraction of isomaltulose in the formulation is 40 to 70% by weight, based on the dry matter of the formulation.

22. Use according to any one of claims 10 to 21, wherein the fraction of isomaltulose in the formulation is 50 to 61 % by weight, based on the dry matter of the formulation.

23. Use according to any one of claims 10 to 22, wherein the formulation is an instant powder, a dry flavour formula, a drink powder, a food, a luxury food or a pharmaceutical.

24. Use according to any one of claims 10 to 23, wherein the formulation contains food-compatible acids, food-compatible salts, intense sweeteners, taste substances, flavour or odour substances, a further sweetening agent and/or caffeine.

25. Use according to any one of claims 10 to 24, wherein the fraction of food-compatible acids in the formulation is 5 to 10% by weight, based on the dry matter of the formulation.

26. Use according to any one of claims 10 to 25, wherein the fraction of intense sweeteners in the formulation is 0.1 to 1% by weight, based on the dry matter of the formulation.

27. Use according to any one of claims 10 to 26, wherein the fraction of taste substances in the formulation is 0.5 to 30% by weight, based on the dry matter of the formulation.

28. Use according to any one of claims 10 to 27, wherein the formulation is in dry form.

29. Use according to any one of claims 10 to 28, wherein the formulation is in freeze-dried, agglomerated or pulverized form.

30. Use according to any one of claims 10 to 29, wherein the formulation contains flow enhancers, in particular silica.

31. Use according to any one of claims 10 to 30, wherein the formulation contains natural and/or synthetic dyes.

32. Use according to any one of claims 10 to 31, wherein the formulation contains natural or synthetic vitamins.

33. Use according to any one of claims 10 to 32, wherein the formulation contains minerals and trace elements.

34. Instant powder containing 50 to 80% by weight isomaltulose, 0.1 to 10% by weight EGCG, 0.1 to 10% by weight caffeine, and also 0.005 to 1.0% by weight B vitamins.

## Revendications

1. Préparation contenant 0,1 à 10 % en poids de composition contenant du polyphénol (par rapport à la substance sèche de la préparation) avec une proportion d'au moins 90 % en poids d'épigallocatéchine gallate (EGCG), par rapport à la substance sèche de la composition contenant du polyphénol, et 30 à 80 % en poids d'isomaltulose, par rapport à la substance sèche de la préparation.

2. Préparation selon la revendication 1, dans laquelle la composition contenant du polyphénol présente une proportion d'au moins 94 % en poids d'EGCG, par rapport au poids de la substance sèche de la composition contenant du polyphénol.

3. Préparation selon la revendication 1 ou 2, dans laquelle la composition contenant du polyphénol présente une proportion de 2,5 % en poids maximum de caféine, de préférence de 0,1 % en poids maximum, à chaque fois par rapport au poids de la substance sèche de la composition contenant du polyphénol.

4. Préparation selon une des revendications précédentes, dans laquelle la composition contenant du polyphénol présente une proportion de 2,5 % en poids maximum de gallocatéchine gallate (GCG), par rapport au poids de la substance sèche de la composition contenant du polyphénol.

5. Préparation selon une des revendications précédentes, dans laquelle a composition contenant du polyphénol présente une proportion de 5,0 % en poids maximum d'épicatéchine gallate (ECG), de préférence de 3,0 % en poids maximum, à chaque fois par rapport au poids de la substance sèche de la composition contenant du polyphénol.

6. Préparation selon une des revendications précédentes, dans laquelle la composition contenant du polyphénol présente une proportion de 0,1 % en poids maximum d'acide gallique, par rapport au poids de la substance sèche de la composition contenant du polyphénol.

7. Préparation selon une des revendications précédentes, dans laquelle la composition contenant du polyphénol présente, à part de l'EGCG, une proportion de 5,0 % en poids maximum d'autres polyphénols et catéchines tels que le gallocatéchine gallate (GCG), le catéchine gallate (CG), l'épicatéchine gallate (ECG), l'épigallocatéchine (EGC), la gallocatéchine (GC) et l'épicatéchine (EC), par rapport au poids de la substance sèche de la composition contenant du polyphénol.

8. Préparation selon une des revendications précédentes, dans laquelle la préparation présente, outre l'isomaltulose, au moins un glucide fortement édulcorant.

9. Préparation selon la revendication 8, dans laquelle la proportion de l'isomaltulose et du glucide fortement édulcorant dans la préparation est de 30 à 85 % en poids, par rapport au poids de la substance sèche de la préparation.

10. Utilisation d'une préparation selon une ou plusieurs des revendications 1 à 9 pour l'augmentation du brûlage des graisses chez les mammifères, notamment chez l'homme.

11. Utilisation d'une préparation selon une ou plusieurs des revendications 1 à 9 pour le soutien du métabolisme des graisses chez les mammifères, notamment chez l'homme.

12. Utilisation d'une préparation selon une ou plusieurs des revendications 1 à 9 pour la réduction de la masse graisseuse chez les mammifères, notamment chez l'homme.

13. Utilisation d'isomaltulose dans une préparation contenant une composition contenant du polyphénol, notamment une préparation selon une des revendications 1 à 9 pour le masquage du goût.

14. Utilisation selon la revendication 13, dans laquelle le masquage du goût est un masquage du goût d'une composition contenant du polyphénol.

15. Utilisation selon une des revendications 10 à 14, dans laquelle la composition contenant du polyphénol est un extrait végétal.

16. Utilisation selon une des revendications 10 à 15, dans laquelle l'extrait végétal est un extrait de thé noir, un extrait de thé Oolong ou un extrait de thé vert.

17. Utilisation selon une des revendications 10 à 16, dans laquelle l'extrait contient des substances odorantes et/ou aromatisantes naturelles ou identiques à celles naturelles supplémentaires.

18. Utilisation selon une des revendications 10 à 17, dans laquelle la composition contenant du polyphénol est l'épigallocatéchine gallate (EGCG).

19. Utilisation selon une des revendications 10 à 18, dans laquelle l'EGCG dans la composition contenant du polyphénol est présent en une proportion d'au moins 94 % en poids, à chaque fois par rapport à la substance sèche de la composition contenant du polyphénol.

20. Utilisation selon une des revendications 10 à 19, dans laquelle la proportion de la composition contenant du polyphénol dans la préparation est de 0,3 à 5 % en poids, par rapport à la substance sèche de la préparation.

21. Utilisation selon une des revendications 10 à 20, dans laquelle la proportion de l'isomaltulose dans la préparation est de 40 à 70 % en poids, par rapport à la substance sèche de la préparation.

22. Utilisation selon une des revendications 10 à 21, dans laquelle la proportion de l'isomaltulose dans la préparation est de 50 à 61 % en poids, par rapport à la substance sèche de la préparation.

23. Utilisation selon une des revendications 10 à 22, dans laquelle la préparation est une poudre instantanée, une formulation aromatisante sèche, une poudre pour boisson, un aliment, une denrée de luxe ou un médicament.

24. Utilisation selon une des revendications 10 à 23, dans laquelle la préparation contient des acides alimentaires, des sels alimentaires, des édulcorants intenses, des substances aromatisantes, des arômes ou des substances odorantes, un autre agent édulcorant et/ou de la caféine.

25. Utilisation selon une des revendications 10 à 24, dans laquelle la proportion des acides alimentaires dans la préparation est de 5 à 10 % en poids, par rapport à la substance sèche de la préparation.

26. Utilisation selon une des revendications 10 à 25, dans laquelle la proportion des édulcorants intenses dans la préparation est de 0,1 à 1 % en poids, par rapport à la substance sèche de la préparation.

27. Utilisation selon une des revendications 10 à 26, dans laquelle la proportion des substances aromatisantes dans la préparation est de 0,5 à 30 % en poids, par rapport à la substance sèche de la préparation.

28. Utilisation selon une des revendications 10 à 27, dans laquelle la préparation est présente sous forme sèche.

29. Utilisation selon une des revendications 10 à 28, dans laquelle la préparation est présente sous forme lyophilisée, agglomérée ou pulvérisée.

30. Utilisation selon une des revendications 10 à 29, dans laquelle la préparation contient des adjuvants d'écoulement, notamment du dioxyde de silicium.

31. Utilisation selon une des revendications 10 à 30, dans laquelle la préparation contient des colorants naturels et/ou synthétiques.

32. Utilisation selon une des revendications 10 à 31, dans laquelle la préparation contient des vitamines naturelles ou synthétiques.

33. Utilisation selon une des revendications 10 à 32, dans laquelle la préparation contient des minéraux et oligoéléments.

34. Poudre instantanée contenant 50 à 80 % en poids d'isomaltulose, 0,1 à 10 % en poids d'EGCG, 0,1 à 10 % en poids de caféine ainsi que 0,005 à 1,0 % en poids de vitamines B.
